# EUROPÄISCHE PATENTSCHRIFT

(11) **EP 1 061 995 B1**
(45) Veröffentlichungstag und Bekanntmachung des Hinweises auf die Patenterteilung: **28.05.2003**
(21) Anmeldenummer: 99913232.7
(22) Anmeldetag: 05.03.1999
(51) Int. Cl.: A61N 1/08, A61N 1/37

(54) **TESTKABELANORDNUNG**
TEST CABLE ARRANGEMENT
CONFIGURATION DE CABLE D'ESSAI

(30) Priorität: 10.03.1998 DE 19810262
(43) Veröffentlichungstag der Anmeldung: 27.12.2000
(73) Patentinhaber: Bisping, Hans-Jürgen, Dipl.-Ing., D-52072 Aachen (DE)
(72) Erfinder: Bisping, Hans-Jürgen, Dipl.-Ing., D-52072 Aachen (DE)
(74) Vertreter: Nau, Walter, Dipl.-Ing.
(86) Internationale Anmeldenummer: EP9901441
(87) Internationale Veröffentlichungsnummer: WO99046000

(56) Entgegenhaltungen:
- DE-U1- 9 003 222
- US-A- 4 141 367
- US-A- 4 705 042
- US-A- 5 411 539
- US-A- 5 679 022

## Beschreibung

Die Erfindung betrifft eine Testkabelanordnung, wie sie bei der Bestimmung von elektrophysiologischen Parametern, z.B. in der Elektrotherapie und Elektrodiagnostik verwendet wird.
Üblicherweise werden bei der Implantation von Herzschrittmachern, Defibrillatoren, Neurostimulatoren und ähnlichen Implantaten oder bei der elektrophysiologischen Untersuchung zunächst die notwendigen Elektroden bzw. Sonden in das Gefäßsystem eingeführt. Wie im US-Patent 4,141,367 beschrieben, wird mit Hilfe eines geeigneten Testkabels eine Verbindung zwischen der sterilen Sonde einerseits und dem im unsterilen Bereich befindlichen Testgerät andererseits hergestellt. Dazu wird eine sterile Testkabelanordnung verwendet.
Das dabei eingesetzte resterilisierbare Testkabel ist massiv gebaut und mit soliden Klemmen ausgestattet Dementsprechend hoch ist auch ihr Gewicht bzw. gering ihre Flexibilität. Diese Eigenschaften lassen es nicht zu, dass das Testkabel während der Manipulation der Sonde angeklemmt bleibt. Weiterhin ist es schwierig, die Krokodilklemmen an dem kleinen Stecker der Sonde so zu befestigen, dass eine hinreichend sichere Verbindung hergestellt wird, ohne dass elektrische Kontaktfehler entstehen. Nach Durchführung der Messungen wird dann das Messkabel gereinigt, getrocknet, auf elektrische und mechanische Unversehrtheit hin überprüft, verpackt und abschließend zur Sterilisation gegeben. Abgesehen von dem logistischen Problem, zur rechten Zeit ein steriles Kabel zur Verfügung zu haben, ergibt sich noch ein anderes Problem:
Da die Überprüfung des Kabels üblicherweise vom Pflegepersonal ohne entsprechende Anweisungen und Testeinrichtungen vorgenommen wird, ist eine Qualitätskontrolle kaum gewährleistet. Kabeibrüche, Isolationsdefekte, Wackelkontakte oder hygienische Mängel können die Folge sein.

Der Erfindung liegt die Aufgabe zugrunde, eine Testkabelanordnung bereit zu stellen, die die genannten Nachteile vermeidet, und ein Testkabel so auszubilden, dass die notwendige gleichbleibende Qualität, Sterilität und ständige Verfügbarkeit gewährleistet. Es soll ein guter Kontakt zum Stecker der Sonde gewährleistet und das Testkabel einfach zu handhaben sein.

Diese Aufgabe wird durch die kennzeichnenden Merkmale des Anspruchs 1 gelöst.

Das Testkabel weist auf der Sondenseite, also im sterilen Bereich ein Trägerelement mit darauf angeordneten Kontaktelementen auf, wobei das Trägerelement vorzugsweise eine ca. 6cm x 8cm große Kartonkarte ist. An diesem Karton befestigt ist eine röhrchenförmige Kontaktfeder mit einer Öffnung, in die der Kontaktstift eines nach EN 50077 hergestellten Elektrodensteckers - der üblicherweise auch als IS-1 Stecker bezeichnet wird - gesteckt wird.

Zur Gewährleistung eines zuverlässigen elektrischen und mechanischen Kontaktes ist die Kontaktfeder mit elastischen Elementen ausgestattet oder weist wie im vorliegenden Fall, eine mechanische Vorspannung des geschlitzten Röhrchens auf.

Der zweite, für den Messvorgang bei bipolaren Sonden benötigte elektrische Anschluss mit dem IS-1 Elektrodensteckerring wird über eine Krokodilklemme hergestellt. Kontaktfeder und Krokodilklemme sind jeweils mit einer dünnen Zwillingslitze, z.B. LIY (Z) mit 0.08mm2 Drahtquerschnitt und Außendurchmesser von 0,9 mm je Strang verbunden.

Der bandförmige Teil der Kontaktfeder kann durch zwei Schlitze in dem Trägerelement hindurch gesteckt und dadurch zuverlässig auf ihm fixiert sein. Die Kontaktfeder kann aber auch auf beliebige andere Art und Weise, wie z.B. durch Nieten, Kleben oder durch andere Befestigungsarten auf dem Trägerelement befestigt sein.

Die Kontaktfeder ist gemäß einer weiteren Ausgestaltung der Erfindung an dem der Einstecköffnung gegenüberliegenden Ende des Rohrteiles mit einer trichterförmigen Aufweitung und einem Führungskanal versehen, welche zusammen das Einführen eines Mandrins in den IS-1 Stecker erleichtern.

Das Trägerelement weist weiterhin an mindestens einer Außenkante Führungsnuten oder Aussparungen auf, die die Litze, wenn sie im Lager- oder Transportzustand um das Trägerelement herum gewickelt ist, am Herunterrutschen hindert.

An geeigneter Stelle ist die Kontaktfeder mit einem Strang der Leitungslitze verlötet oder in anderer elektrischer Wirkverbindung, z.B. durch Krimpen mit ihm verbunden. Der andere Strang der Zwillingslitze ist elektrisch mit der Krokodilklemme verbunden.
Es dient als Zugentlastung, dass die Zwillingslitze mit ihren Einzelsträngen oder als Ganzes durch Ausstanzungen in dem Trägerelement hindurchgesteckt ist.

Die Länge des mit der Krokodilklemme verbundenen freien Stranges der Zwillingslitze ist um einige Zentimeter größer als die des mit der Kontaktfeder verbundenen Stranges. Dadurch wird erreicht, dass die Krokodilklemme noch ausreichenden Bewegungsspielraum zum Anklemmen an den IS-1 Anschluss hat.

Handelt es sich bei dem zu testenden Sondensystem um eine sog. unipolare Konfiguration, zieht man die Krokodilklemme einfach von dem Trägerelement ab, verlängert die freie Länge dieses Litzenstranges durch Auftrennen der Zwillingsverbindung auf der notwendigen Länge und bringt die Krokodilklemme mit dem Gewebe in bekannter Weise in elektrischen Kontakt.

Das Trägerelement ist aus einem Material, z.B. Pappe, hergestellt, weiches durch seine Beschaffenheit sicherstellt, dass es nur einmalig verwendet werden kann. Säubern durch Abwaschen und anschließendes Resterilisieren ist bei Pappe bzw. Pappkarton nicht möglich. Damit wird sichergestellt, daß das gesamte Testkabel nicht wiederverwendet werden kann und somit für jeden Messvorgang ein neues, qualitätskontrolliertes Testkabelsystem verwendet werden muss.

Man kann leicht nachvollziehen, dass erfindungsgemäße Testkabel für den einmaligen Gebrauch durch Massenherstellung letztendlich preiswerter sind, wenn man die Kosten für Säuberung, Verpackung und Resterilisation von üblichen, bisher verwendeten Messkabeln mit all ihrem Zeit- und Personalaufwand berücksichtigt.

Das andere proximale Ende der elektrischen Leitung in Form der Zwillingslitze liegt i.d.R. im unsterilen Bereich und muss letztlich mit der Testeinrichtung verbunden werden.

Dieses Kabelterminal muss folgenden Anforderungen genügen: Zuverlässiger elektrischer Kontakt mit der Testeinrichtung sowie Schutz des Patienten vor gefährlichen Spannungen oder Strömen.

Es werden erfindungsgemäß folgende Lösungen vorgeschlagen:
Die Zwillingslitze ist an einem zweiten proximalen Trägerelement z.B. aus Pappe befestigt und ihre elektrischen Leiter sind mit Lötösen verbunden, die an getrennten Orten, möglichst jedoch am Rand des Trägerelementes angeordnet sind. Auch hierbei sind Niet-, Klebe- oder die bereits oben beschriebenen Durchsteckbefestigungen vorgesehen.
Zur Kontaktierung mit dem Testgerät wird nun das Originalanschlusskabel des Testgerätes verwendet, welches in diesem Fall nicht steril zu sein braucht und daher nicht der belastenden Reinigungs-/Sterilisationsprozedur unterzogen werden muss.
Da diese Testkabel i.d.R. mit 2 Krokodilklemmen ausgestattet sind, bieten sich die Ringe der Lötösen gut als Befestigungsort an.
Auch wäre es denkbar, anstelle der Lötösen am Ende jedes Leitungsstranges die abisolierten Drahtenden zu Schlaufen zu biegen und zusammenzulöten. In diese Drahtschlaufen könnten dann die Krokodilklemmen des Messkabels eingreifen. Die o.g. Ösen oder Drahtschlaufen sollen ein Herausrutschen der Krokodilklemme von der Drahtlitze verhindern.

Eine andere Alternative zur Kontaktierung der Litze mit dem Testgerät besteht darin, dass die Litzenleitungen einfach abgetrennt sind und isoliert enden. Erst durch Einstecken in entsprechende Terminals und durch durch die Isolierung hindurchschneidende Kontaktmesser wird der notwendige elektrische Kontakt erzielt. Derartige Kontaktierungen sind z.B. bei RJ11 Steckern, den sog. Westernsteckern aus der Telefontechnik, üblich.

Es wird weiter vorgeschlagen, dass am proximalen Ende der Litze, die vorzugsweise einen Strangdurchmesser von weniger als 0,95 mm aufweist. eine Steckverbindung z.B. in Form eines RJ11-4 Steckers angeordnet ist, welcher in eine passende Buchse des Testgerätes oder eines Anschlusskabels gesteckt wird. Um bereits im Einsatz befindliche Testgeräte nicht modifizieren zu müssen, wird vorgeschlagen, dass das zum Testgerät gehörige original Testkabel an der Patientenseite, also dort, wo die Krokodilklemmen angeordnet sind , stattdessen mit einer passenden Buchse versehen wird. Diese als Westernstecker aus der Telefontechnik bekannten RJ11-Steckverbindungen haben folgende Vorteile: sie sind preiswert, lassen sich mit der Litzenleitung einfach verbinden und weisen einen Berührungsschutz auf. Verwendet man Stecker vom Typ RJ11-4, so sind sie, da sie für den Höreranschluss konzipiert wurden, nicht in Telefondosen einsteckbar.

Zur besseren Unterscheidung soll die Zwillingslitze Leitungsstränge in unterschiedlichen Farben aufweisen.

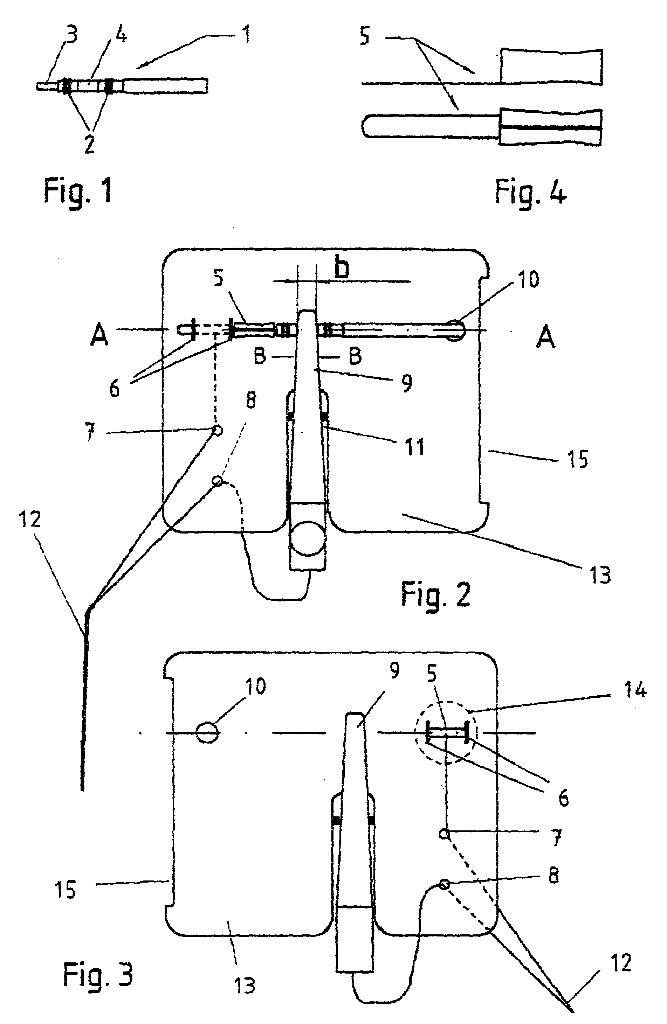

Das Loch in dem Trägerelement nach Fig. 2 dient zur Führung der Sonde. Steckt man nämlich die Sonde zunächst von unten durch dieses Elektrodensteckloch und stellt danach die Verbindung des IS1 Kontaktstiftes mit der Kontaktfeder her, so ist der IS-1 Anschluss in der Achse AA fixiert.
An der proximalen Trägerplatte ist ebenfalls ein ca. 5 mm großes Loch eingestanzt, welches hier jedoch dazu dient, die Trägerplatte z.B. mit Hilfe einer Tuchklemme zu fixieren, um ein Herunterrutschen zu verhindern, wenn das Kabel des Testgerätes angeschlossen wird.

Ein anderes Merkmal ist das geringe Gewicht und die hohe Flexibilität der Anordnung. Bei der erfindungsgemäßen Anordnung ist die Masse der Litzen nur etwa in der Größenordnung von etwa 3g/m (Zwillingslitze) bei einem Litzenstrangdurchmesser von ca. 0,9 mm. Die Masse einer 100 cm langen Testkabelanordnung mit proximalem Trägerelement beträgt nur etwa 9g. Da diese dünne, leichte und flexible Litze geringste Biege- und Torsionskräfte erfordert und damit kaum behindert, können die Kontaktierungen während der gesamten Plazierungsprozedur der Sonde bestehen bleiben.

Dadurch, dass das Testkabel aus dünnlitziger, leichter und flexibler Leitungslitze hergestellt und als Wegwerfteil ausgebildet ist, wird dauerhaft Qualität und Sterilität gewährleistet.

Werden die Litzen an den Krokodilklemmen oder Kontaktfedern angelötet, so ist nicht auszuschließen, dass an den Lötstellen spitze und scharfe Kanten entstehen, die einen OP-Handschuh u.U. beschädigen können. Es wird daher vorgeschlagen, dass über die Lötstellen Klebepunkte aus Papier oder anderem Material befestigt werden.
Es wird auch vorgeschlagen, dass die Lötstellen an der dem Trägerelement zugewandten Seite angeordnet sind .
Weiterhin wird vorgeschlagen, dass das proximale Trägerelement zwei elektrisch getrennte Kontaktflächen aufweist, z.B. in Form von Kupferbeschichtungen auf einer Kunststoffplatte, weiche mit den entsprechenden Leitungssträngen elektrisch verbunden sind. Diese Kontaktkarte mit den Abmessungen etwa einer Scheckkarte oder kleiner wird dann in einen Schlitz in das Testgerät oder eines angeschlossenen Adapters eingesteckt und verbindet die Litzen mit dem Testgerät.

Kommen sog. Zweikammerschrittmacher zum Einsatz, so werden zwei Elektroden implantiert, und zwar eine im Vorhof und eine im Ventrikel.
Geeignete Testgeräte haben dann einen Anschluss sowohl für die Vorhofals auch für die Ventrielektrode. Dementsprechend sind auch zwei getrennte Messkabel erforderlich.
Es wird für diesen Einsatzzweck vorgeschlagen, dass z.B. ein Westernstecker (auch als Modularstecker RJ11/RJ12/RJ45 bekannt) mit zwei Zwillingslitzen verbunden ist, die an ihrem distalen Ende jeweils ein Trägerelement mit den notwendigen Kontaktmitteln nach bereits oben beschriebener Konfiguration aufweisen und entsprechende Unterscheidungsmerkmale für die Verwendung an der Vorhof- bzw. Ventrikelektrode tragen.

Es wird weiterhin vorgeschlagen, dass die Kontaktbeiegung des modularen Anschlusssteckers -22- in diesem Fall so vorgesehen ist, dass an ein Zweikammertestgerät über die Anschlussbuchse -23-, sowohl eine erfindungsgemäße Testkabelanordnung für Einkammeranwendung als auch für Zweikammeranwendung anschließbar ist
Denkbar ist z.B. eine Anordnung, bei der die Zwillingslitze für die Vorhofelektrode an die nebeneinanderliegenden Kontakte -1- und -2- und die Zwillingslitze für die Ventrikelektrode an die Kontakte -3- und -4- angeschlossen wird.
Ein erfindungsgemäßes Testkabel für eine Einkammeranwendung im Ventrikel würde dann nur die Kontaktbelegung -3- und -4- aufweisen.

Weitere vorteilhafte Ausgestaltungen der Erfindung sind der Zeichnungsbeschreibung zu entnehmen, in der die Ausführungsbeispiele der Erfindung näher beschrieben sind.

Es zeigen:
- Fig. 1:: Eine Längsansicht eines IS-1 Anschlusssteckers
- Fig. 2:: Eine Ansicht eines Trägerelementes
- Fig. 3:: Die rückseitige Ansicht des Trägerelementes nach Fig. 2
- Fig. 4:: Drauf- und Seitenansicht einer Kontaktfeder
- Fig. 5:: Eine Ansicht eines proximalen Trägerelementes
- Fig. 6:: Schematische Darstellung einer Testkabelanordnung, bei der die proximale Verbindung als Stecker ausgeführt ist.
- Fig. 7:: Seitenansicht einer Klammerklemme, die nicht Bestandteil der Erfindung ist

Ein in der Schrittmacher- und Defibrillatortherapie seit Jahren eingeführter Elektrodenstecker 1 nach dem sog. IS-1 Standard ist in Fig. 1 dargestellt. Mit 3 ist der vordere Kontaktstift des IS-1 Steckers und mit 4 der zurückliegende Elektrodensteckerring bezeichnet. Die Dichtringe 2 sind aus isolierendem Silikongummi oder Polyurethan (PU) hergestellt und begrenzen den Bereich des Elektrodensteckerringes 4.

Fig. 2 stellt in etwa maßstabsgetreu den Teil der Testkabelanordnung dar, der im sterilen Bereich liegt und den elektrischen Kontakt mit der Sonde bzw. Elektrode herstellt.
Zunächst ist die Sonde mit ihrem Stecker 1 von unten durch ein ca. 5mm großes Loch 10 in dem Trägerelement 13 hindurchgesteckt worden und danach der Kontaktstift 3 in die röhrenförmige Öffnung der Kontaktfeder 5 eingeführt worden.

In der Lager- und Transportphase ist die Krokodilklemme 9 dergestalt in dem Führungskanal 11 angeordnet, daß ihre Greifschenkel mit ihren vorderen Enden mit der Linie BB abschließen und sich in dem Trägerelement 13 fixieren. Nach dem Befestigen des IS-1 Steckers 1 - wie oben erwähnt - wird dann die Krokodilklemme 9 mit ihren Greifschenkeln so plaziert, daß diese genau über dem Elektrodenring 4 zu liegen kommt und ein Schenkel elektrischen Kontakt mit dem Elektrodenring 4 herstellt. Die geometrische Anordnung, vor allen Dingen des Führungskanals 11, ist dabei so gewählt, daß der kontaktierende Schenkel der Krokodilklemme 9 genau über dem Elektrodensteckerring 4 zu liegen kommt und nicht auf einem oder mehreren Dichtringen 2 .

Der Führungskanal 11 begrenzt dabei die Beweglichkeit der Krokodilklemme 9 nicht nur in seitlicher Richtung sondern auch in der Vorschieberichtung. Die Breite b des Greifschenkels, dort, wo er mit dem Elektrodensteckerring 4 in Kontakt kommt, ist kleiner als die Breite des Elektrodensteckerrings 4 gewählt.

Fig. 7 stellt schematisch eine Klammerklemme 26 dar, die an ihrem unteren Ende Blechfahnen zum Befestigen trägt, wobei dieses Ausführungsbeispiel nicht Bestandteil der Erfindung ist.

In Figur 2 und 3 erkennt man weiterhin eine Aussparung 15 in dem Trägerelement 13, welche dazu dient, bei Lagerung und Transport die aufgewickelte Leitungslitze 12 vor dem Herunterrutschen zu bewahren. Zwei in das Trägerelement 13 eingestanzte Schlitze 6 dienen zur Befestigung der Kontaktfeder 5. Diese wird gemäß der Zeichnung durch einfaches Durchstecken sicher in der gewünschten Position gehalten. Die Ausstanzungen 7/8 dienen dazu, die Litzenstränge zu führen und eine gewisse Zugentlastung zu gewährleisten.
Der Klebepunkt 14 über der Lötstelle, die einen Litzenstrang mit der Klemmfeder 5 verbindet, hat die Aufgabe, eventuell vorhandene scharfe oder spitze Lötstellen abzudecken, um zu verhindern, daß die OP-Handschuhe des Anwenders beschädigt werden.

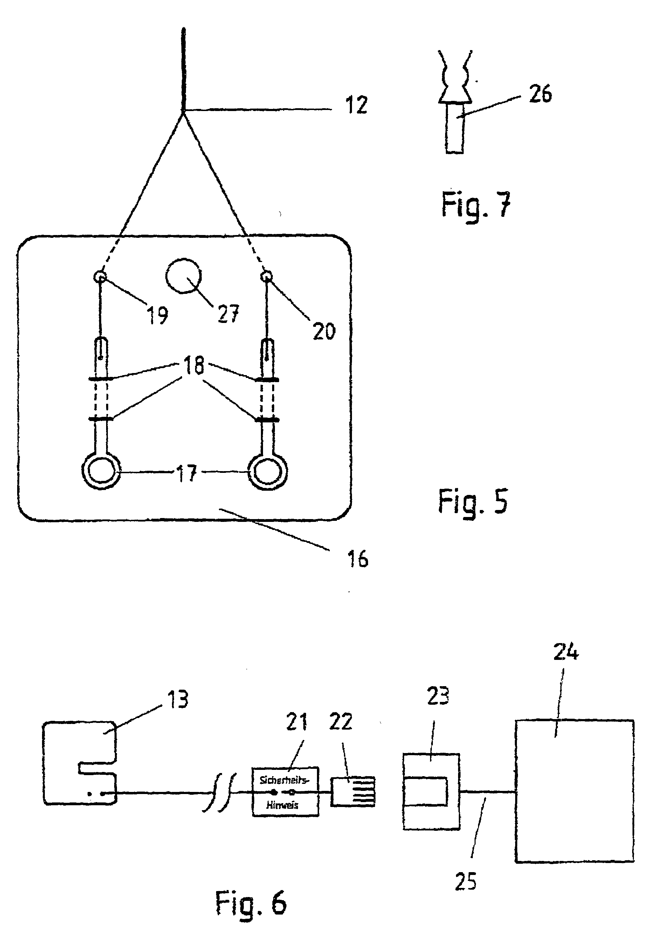

In Fig. 5 ist das proximale, also im unsterilen Bereich liegende Ende des Testkabels dargestellt. Auch hier ist wieder ein proximales Trägerelement 16 aus Pappe vorhanden, welches die Löcher 19 und 20, sowie die Schlitze 18 enthält. Die Löcher 19 und 20 dienen auch wiederum als Fixationspunkte und Zugentlastung für die Litzenleitung, die Schlitze 18 dienen als Befestigung für die durch sie hindurchgesteckten Lötösen 17. Diese sind mit je einem Litzenstrang verlötet und weisen entweder eine Abdeckung aus einem Klebepunkt auf oder sind auf der Unterseite der Lötöse 17 angelötet, sodaß eventuell scharfe Teile keinen Schaden anrichten können. Die Verlötung könnte dabei so zu denken sein, daß zunächst die Lötöse 17 in die entsprechenden Schlitze 18 eingesteckt wird, das freie Ende der Lötöse 17 senkrecht hochgebogen wird, dann die Lötung auf der "Unterseite" durchgeführt wird und anschließend die Lötösenfahne wieder zurückgebogen wird. Das ca 5 mm große Loch 27 dient zur Fixierung der proximalen Trägerplatte mit Hilfe einer sog. Tuchklemme.

Ganz ohne das proximale Trägerelement 16 kommt man mit dem erfindungsgemäßen Vorschlag nach Fig. 6 aus. Dort befindet sich nämlich am Ende der Litze eine Steckvorrichtung 22, z.B. in Form eines Westernsteckers RJ11/4, welcher in eine passende Buchse 23 einsteckbar ist. Diese wiederum steht über ein Kabel 25 mit dem Testgerät 24 in elektrischer Wirkverbindung.
Eine Pappkarte 21 ist mit der Zwillingslitze verbunden und trägt für die Benutzung bedeutsame Sicherheitshinweise.

## Patentansprüche

1. Testkabel in mindestens zweiadriger Ausführung für den sterilen Einsatz, z.B. bei Schrittmacherimplantationen oder bei der elektrophysiologischen Untersuchung, das als elektrische Verbindung zwischen einem Elektrodenstecker (1) einer Sonde und einem Testgerät (24) dient und dem Testgerät oder einem Anschlusskabel des Testgerätes zugeordnete elektrische Verbindungselemente sowie an einen Kontaktstift (3) und einen Elektrodensteckerring (4) des Elektrodensteckers (1) anschließbare Kontaktelemente aufweist wobei der Bereich des Elektrodensteckerrings (4) von Dichtringen (2) begrenzt wird, und wobei das mit dem Elektrodensteckerring (4) zu verbindende Kontaktelement als Krokodilklemme (9) aus geführt ist,
**dadurch gekennzeichnet, dass** das Testkabel eine dünnlitzige flexible Leitungslitze (12) und an seinem mit dem Elektrodenstecker (1) der Sonde zu verbindenden Ende ein Trägerelement (13) aufweist, sowie als Wegwerfteil ausgebildet ist,
dass das mit dem Kontaktstift (3) zu verbindende Kontaktelement als Kontaktfeder (5) oder Kontakthülse ausgebildet ist, die am Trägerelement (13) befestigt ist,
wobei in dem Trägerelement (13) ein Führungskanal (11) für die Krokodilklemme (9) so angeordnet ist, dass die Krokodilklemme (9) so platzierbar ist, dass diese genan über dem Elektrodensteckerring (4) zu Liegen Kommt und ein Schenkel der Krokodilklemme (9) elektrischen Kontakt mit dem Elektrodensteckerring (4) herstellt, wobei die geometrische Anordnung des Führungskanals (11) so gewählt ist, dass der Kontaktierende Schenkel der Krokodilklemme (9) genan über dem Elektrodensteckerring (4) zu Liegen Kommt und nicht auf einem oder mehreren Dichtringen (2), wobei der Führungskanal (11) die Beweglichkeit der Krokodilklemme (9) nicht nur in seitlicher Richtung sondern auch in der Vorschieberichtung begrenzt.

2. Testkabel nach Anspruch 1,
**dadurch gekennzeichnet, dass** die Kontaktfeder (5) oder Kontakthülse den Kontaktstift (3) des Elektrodensteckers (1) der Sonde zumindest teilweise umschließt.

3. Testkabel nach einem der Ansprüche 1 oder 2,
**dadurch gekennzeichnet, dass** die Kontaktfeder (5) ein geschlitztes Röhrchen aufweist.

4. Testkabel nach Anspruch 1,
**dadurch gekennzeichnet, dass** das Trägerelement (13) zumindest zwei parallel und zueinander fluchtend angeordnete Schlitze (6) aufweist, in die die Kontaktfeder (5) oder Kontakthülse so eingesteckt ist, dass sie außerhalb des durch die Schlitze (6) definierten Bereichs auf ein und derselben Seite des Trägerelements (13) angeordnet ist.

5. Testkabel nach einem der Ansprüche 1 oder 4,
**dadurch gekennzeichnet, dass** in Verlängerung der Schlitze (6) und zu diesen beabstandet in dem Trägerelement (13) ein Elektrodensteckloch (10) angeordnet ist, so dass nach Durchstecken des Elektrodensteckers (1) durch das Elektrodensteckloch (10) der Kontaktstift (3) in die Kontaktfeder (5) oder Kontakthülse einschiebbar ist.

6. Testkabel nach einem der Ansprüche 4 oder 5,
**dadurch gekennzeichnet, dass** der Führungskanal (11) für die Krokodilklemme (9) quer zu den Schlitzen (6) und dem Elektrodensteckloch (10) in dem Trägerelement (13) angeordnet ist.

7. Testkabel nach Anspruch 1,
**dadurch gekennzeichnet, dass** die dem Testgerät (24) zugeordneten Verbindungselemente an einem proximalen Trägerelement (16) befestigbar sind.

8. Testkabel nach Anspruch 7,
**dadurch gekennzeichnet, dass** das proximale Trägerelement (16) je zwei parallel und zueinander fluchtend angeordnete Schlitze (18) aufweist, in die je eine Lötöse (17) so eingesteckt ist, dass sie außerhalb des durch die Schlitze (18) definierten Bereichs auf ein und derselben Seite des proximalen Trägerelements (16) angeordnet sind.

9. Testkabel nach Anspruch 8,
**dadurch gekennzeichnet, dass** je eine Litze der Leitungslitze (12) an den Lötösen (17) befestigt ist und durch je ein in dem proximalen Trägerelement (16) vorgesehenes Loch (19 und 20) geführt sind.

10. Testkabel nach einem der vorhergehenden Ansprüche,
**dadurch gekennzeichnet, dass** das Trägerelement (13) oder das proximale Trägerelement (16) aus einem flexiblen und einer Reinigung nicht standhaltenden Material, z.B. Pappe, hergestellt ist.

11. Testkabel nach Anspruch 1,
**dadurch gekennzeichnet, dass** die dem Testgerät (24) zugeordneten Verbindungselemente in einem Stecker (22) zusammengefasst sind, der mit einer entsprechenden Dose (23), die mit dem Testgerät (24) in Verbindung steht, verbindbar ist.

12. Testkabel nach einem der vorhergehenden Ansprüche,
**dadurch gekennzeichnet, dass** in einem Stecker (22) zwei Zwillingslitzen zusammengefasst sind.

13. Testkabel nach einem der vorhergehenden Ansprüche,
**dadurch gekennzeichnet, dass** der Drahtquerschnitt eines Stranges der Leitungslitze (12) einen Querschnitt von kleiner gleich 0,1 mm² aufweist.

14. Testkabel nach einem der vorhergehenden Ansprüche,
**dadurch gekennzeichnet, dass** die Isolationshülle eines Stranges der Leitungslitze (12) einen Außendurchmesser von kleiner gleich 0,95 mm aufweist.

15. Testkabel nach einem der vorhergehenden Ansprüche,
**dadurch gekennzeichnet, dass** die Testkabelanordnung bei einer Länge von 150 cm eine Masse von maximal 40 g aufweist.

## Claims

1. Test cable of an at least two-core configuration for sterile use, for example in the case of pacemaker implantations or in the case of electrophysiological examination, which serves as an electrical connection between an electrode connector (1) of a probe and a test device (24) and electrical connecting elements assigned to the test device or to a connection cable of the test device and also contact elements which can be connected to a contact pin (3) and an electrode connector ring (4) of the electrode connector (1), the region of the electrode connector ring (4) being delimited by sealing rings (2), and the contact element which is to be connected to the electrode connector ring (4) being configured as a crocodile clip (9), **characterized in that** the test cable has a thin-stranded flexible pigtail lead (12) and, at its end which is to be connected to the electrode connector (1) of the probe, a carrier element (13), and is designed as a disposable part, **in that** the contact element which is to be connected to the contact pin (3) is designed as a contact spring (5) or contact sleeve which is fastened to the carrier element (13), a guide channel (11) for the crocodile clip (9) being arranged in the carrier element (13) in such a way that the crocodile clip (9) can be placed in such a way that it comes to lie exactly over the electrode connector ring (4) and one leg of the crocodile clip (9) establishes electrical contact with the electrode connector ring (4), the geometrical arrangement of the guide channel (11) being chosen such that the contacting leg of the crocodile clip (9) comes to lie exactly over the electrode connector ring (4) and not on one or more sealing rings (2), the guide channel (11) delimiting the freedom of movement of the crocodile clip (9) not only in the lateral direction but also in the pushing-forward direction.

2. Test cable according to Claim 1, **characterized in that** the contact spring (5) or contact sleeve at least partly encloses the contact pin (3) of the electrode connector (1) of the probe.

3. Test cable according to one of Claims 1 or 2, **characterized in that** the contact spring (5) has a slit tube.

4. Test cable according to Claim 1, **characterized in that** the carrier element (13) has at least two slits (6) which are arranged parallel and in line with each other and into which the contact spring (5) or contact sleeve can be inserted in such a way that it is arranged outside the region defined by the slits (6) on one and the same side of the carrier element (13).

5. Test cable according to one of Claims 1 or 4, **characterized in that**, as an extension of the slits (6) and at a distance from them, an electrode insertion hole (10) is arranged in the carrier element (13), so that, after the electrode connector (1) has been inserted through the electrode insertion hole (10), the contact pin (3) can be pushed into the contact spring (5) or contact sleeve.

6. Test cable according to one of Claims 4 or 5, **characterized in that** the guide channel (11) for the crocodile clip (9) is arranged transversely in relation to the slits (6) and the electrode insertion hole (10) in the carrier element (13).

7. Test cable according to Claim 1, **characterized in that** the connecting elements assigned to the test device (24) can be fastened to a proximal carrier element (16).

8. Test cable according to Claim 7, **characterized in that** the proximal carrier element (16) has in each case two slits (18) which are arranged parallel and in line with each other and into which respective soldering eyes (17) can be inserted in such a way that they are arranged outside the region defined by the slits (18) on one and the same side of the proximal carrier element (16).

9. Test cable according to Claim 8, **characterized in that** respective litz wires of the pigtail leads (12) are fastened to the soldering eyes (17) and led through a respective hole (19 and 20) provided in the proximal carrier element (16).

10. Test cable according to one of the preceding claims, **characterized in that** the carrier element (13) or the proximal carrier element (16) is produced from a flexible material which cannot withstand cleaning, for example paperboard.

11. Test cable according to Claim 1, **characterized in that** the connecting elements assigned to the test device (24) are grouped together in a connector (22), which can be connected to a corresponding socket (23) which is in connection with the test device (24).

12. Test cable according to one of the preceding claims, **characterized in that** two twin litz wires are combined in a connector (22).

13. Test cable according to one of the preceding claims, **characterized in that** the wire cross section of a strand of the pigtail lead (12) has a cross section of less than or equal to 0.1 mm².

14. Test cable according to one of the preceding claims, **characterized in that** the insulating sheath of a strand of the pigtail lead (12) has an outside diameter of less than or equal to 0.95 mm.

15. Test cable according to one of the preceding claims, **characterized in that** the test cable arrangement with a length of 150 cm has a mass of at most 40 g.

## Revendications

1. Câble d'essai à au moins deux brins pour usages stériles, par exemple pour des implantations de prothèses de marche ou pour des examens électrophysiologiques, servant de liaison électrique entre un connecteur d'électrode (1) d'une sonde et un appareil d'essai (24) et comportant des éléments électriques de liaison associés à l'appareil d'essai (24) ou à un câble de raccordement de celui-ci ainsi que des éléments de contact pouvant être raccordés à une broche de contact (3) et à une bague (4) du connecteur d'électrode (1), cette dernière ayant sa zone délimitée par des bagues d'étanchéité (2) et l'élément de contact à relier à la bague de connecteur (4) étant constitué par une pince crocodile (9),
**caractérisé en ce que**
- le câble d'essai est un cordon conducteur (12) flexible, mince, ou un élément jetable, et présente un élément porteur (13) à son extrémité à relier au connecteur d'électrode (1) de la sonde,
- l'élément de contact à relier à la broche de contact (3) est constitué par un ressort de contact (5) ou une douille de contact, fixée à l'élément porteur (13),
- dans l'élément porteur (13) se trouve un canal de guidage (11) pour la pince crocodile (9), disposé de manière que cette pince (9) puisse venir se placer précisément sur la bague (4) du connecteur d'électrode et qu'une branche de la pince crocodile (9) établisse un contact électrique avec la bague (4),
- la disposition géométrique du canal de guidage (11) est choisie de manière que la branche de contact de la pince crocodile (9) vient précisément se placer sur la bague du connecteur d'électrode (4) et non sur une ou plusieurs bagues d'étanchéité (2), et ce canal de guidage (11) limite la mobilité de la pince crocodile (9) non seulement en direction latérale mais aussi selon la direction du coulissement.

2. Câble d'essai selon la revendication 1,
**caractérisé en ce que**
le ressort de contact (5) ou la douille de contact entoure au moins en partie la broche de contact (3) du connecteur d'électrode (1) de la sonde.

3. Câble d'essai selon la revendication 1 ou 2,
**caractérisé en ce que**
le ressort de contact (5) est un petit tube fendu.

4. Câble d'essai selon la revendication 1,
**caractérisé en ce que**
l'élément porteur (13) présente au moins deux fentes (6) parallèles et affleurant l'une vis-à-vis de l'autre, dans lesquelles est emmanché le ressort de contact (5) ou la douille de contact de manière que ce ressort ou cette douille, en dehors de la zone définie par les fentes (6) se trouve d'un seul et même côté de l'élément porteur (13).

5. Câble d'essai selon l'une des revendications 1 à 4,
**caractérisé en ce que**
dans le prolongement des fentes (6) et à une certaine distance de celles-ci, l'élément porteur (13) est percé d'un trou d'enflchage d'électrode (10) de sorte qu'après enfichage du connecteur d'électrode (1) dans le trou (10), la broche de contact (3) peut être introduite par coulissement dans le ressort de contact (5) ou la douille de contact.

6. Câble d'essai selon la revendication 4 ou 5,
**caractérisé en ce que**
le canal de guidage (11) de la pince crocodile (9) est disposé dans l'élément porteur (13) perpendiculairement aux fentes (6) et au trou d'enfichage d'électrode (10).

7. Câble d'essai selon la revendication 1,
**caractérisé en ce que**
les éléments de liaison associés à l'appareil d'essai (24) peuvent être fixés sur un élément porteur (16) proximal.

8. Câble d'essai selon la revendication 7,
**caractérisé en ce que**
l'élément porteur proximal (16) présente, affleurant l'une vis-à-vis de l'autre et parallèles, des fentes (18) dans chacune desquelles est emmanchée une cosse à souder (17) de manière que ces cosses, en dehors de la zone définie par les fentes (18), se trouvent d'un seul et même côté de l'élément porteur (16).

9. Câble d'essai selon la revendication 8,
**caractérisé en ce qu'**
à chacune des cosses à souder (17) est fixé un brin du cordon conducteur (12) et ces brins passent chacun dans le trou correspondant (19 ou 20) prévu dans l'élément porteur (16) proximal.

10. Câble d'essai selon l'une des revendications précédentes,
**caractérisé en ce que**
l'élément porteur (13) ou l'élément porteur proximal (16) est fait d'un matériau ne résistant pas au nettoyage, par exemple du carton.

11. Câble d'essai,
**caractérisé en ce que**
les éléments de liaison associés à l'appareil d'essai (24) sont réunis dans une fiche (22) qui peut être reliée à une prise (23) elle-même reliée à l'appareil d'essai (24).

12. Câble d'essai selon l'une des revendications précédentes,
**caractérisé en ce que**
dans une fiche (22) sont réunis deux cordons jumelés.

13. Câble d'essai selon l'une des revendications précédentes,
**caractérisé en ce que**
la section conductrice des fils d'un brin du cordon conducteur (12) est inférieure à 0,1 mm².

14. Câble d'essai selon l'une des revendications précédentes,
**caractérisé en ce que**
la douille d'isolation d'un brin de cordon conducteur (12) a un diamètre extérieur ne dépassant pas 0,95 mm.

15. Câble d'essai selon l'une des revendications précédentes,
**caractérisé en ce que**
le système de câble d'essai avec une longueur de 150 cm, a un poids ne dépassant pas 40 g.
